# EUROPEAN PATENT SPECIFICATION

(11) **EP 3 549 596 B1**
(45) Date of publication and mention of the grant of the patent: **05.10.2022**
(21) Application number: 17876506.1
(22) Date of filing: 28.08.2017
(51) Int. Cl.: A61K 38/12, A61P 11/00

(54) **USE OF MICROCYSTINS IN PREPARATION OF DRUGS FOR PREVENTING OR TREATING ORGAN AND TISSUE FIBROSIS DISEASES**
VERWENDUNG VON MICROCYSTINEN ZUR HERSTELLUNG VON ARZNEIMITTELN ZUR PRÄVENTION ODER BEHANDLUNG VON ORGAN- UND GEWEBEFIBROSEERKRANKUNGEN
UTILISATION DE MICROCYSTINES DANS LA PRÉPARATION DE MÉDICAMENTS POUR LA PRÉVENTION OU LE TRAITEMENT DE MALADIES DE FIBROSE D'ORGANE ET DE TISSU

(30) Priority: 30.11.2016 CN 201611079521; 09.01.2017 CN 201710014483
(43) Date of publication of application: 09.10.2019
(73) Proprietor: Nanjing University, Jiangsu 210093 (CN)
(72) Inventor: WANG, Yaping, Nanjing, Jiangsu 210093 (CN); XU, Lizhi, Nanjing, Jiangsu 210093 (CN); WANG, Jie, Nanjing, Jiangsu 210093 (CN); ZHAO, Qingya, Nanjing, Jiangsu 210093 (CN)
(74) Representative: Jacobi, Markus Alexander
(86) International application number: PCT/CN2017/099249
(87) International publication number: WO 2018/099148

(56) References cited:
- WO-A1-2009/059425
- CN-A- 102 524 180
- CN-A- 106 620 647
- US-A1- 2014 271 923
- WANG CONG ET AL: "The toxic effects of microcystin-LR on mouse lungs and alveolar type II epithelial cells", TOXICON, vol. 115, 16 March 2016 (2016-03-16), pages 81-88, XP029499654, ISSN: 0041-0101, DOI: 10.1016/J.TOXICON.2016.03.007
- LOWE J ET AL: "Single sublethal dose of microcystin-LR is responsible for different alterations in biochemical, histological and physiological renal parameters", TOXICON, ELMSFORD, NY, US, vol. 59, no. 6, 7 February 2012 (2012-02-07), pages 601-609, XP028475774, ISSN: 0041-0101, DOI: 10.1016/J.TOXICON.2012.02.003 [retrieved on 2012-02-23]
- DING, XIAOSHENG: "Research Progress on Microcystin and Its Kidney Toxicity", Journal of Anhui Agricultural Sciences, vol. 36, no. 31, 31 December 2008 (2008-12-31), pages 13506-13507, XP9515681,
- LI, XIAOYU et al.: "Research Progress on Relationship between Microcystin and Human Health", Chinese Journal of Public Health, vol. 24, no. 08, 31 August 2008 (2008-08-31), pages 1016-1017, XP009515845, ISSN: 1001-0580
- GUZMAN, R.E. et al.: "Hepatic Oxidative Stress Following Prolonged Sublethal Microcystin LR Exposure", Toxicologic Pathology, vol. 27, no. 5 31 December 1999 (1999-12-31), pages 582-588, XP055490319, Retrieved from the Internet: URL:DOI: 10.1177/019262339902700512
- Loyda Atencio ET AL: "Acute Effects of Microcystins MC-LR and MC-RR on Acid and Alkaline Phosphatase Activities and Pathological Changes in Intraperitoneally Exposed Tilapia Fish ( Oreochromis sp. )", TOXICOLOGIC PATHOLOGY., vol. 36, no. 3, 1 April 2008 (2008-04-01), pages 449-458, XP055682208, US ISSN: 0192-6233, DOI: 10.1177/0192623308315356
- A Milutinovic ET AL: "Microcystin-LR Induces Alterations in Heart Muscle", Folia Biol (Praha). 2006;52(4), 12 December 2006 (2006-12-12), pages 116-118, XP055682003, Retrieved from the Internet: URL:https://fb.cuni.cz/Data/files/folia_bi ologica/volume_52_2006_4/FB2006A0015.pdf [retrieved on 2020-04-01]

## Description

### FIELD OF THE DISCLOSURE

This disclosure relates to the field of medical technology. Disclosed is using microcystins (MCs) in the preparation of drugs for prevention or treatment of fibrosis diseases in human organs and tissues. The scope of the invention is defined by the claims.

### BACKGROUND OF THE DISCLOSURE

Fibrosis is a pathological response of organs and tissues to injury. Organ and tissue fibrosis are characterized by excess deposition of extracellular matrix (ECM), mainly collagen. A similar pathological process presents in the fibrosis diseases of different tissues and organs, involving recurrent and persistent tissue damages caused by exogenous and endogenous inducers, increases in reactive oxygen species (ROS), infiltration of inflammatory cells, release of transforming growth factor-beta (TGF-β), activation of TGF-β/Smad signaling pathway and then myofibroblast differentiation and proliferation. (1-3) Fibrosis generally develops progressively, eventually leading to tissue and organ failure with poor clinical prognosis. (4) Pulmonary fibrosis is a chronic interstitial respiratory disease, in which fibrous scars form and develop in the lung tissues, leading to serious breathing problems. Pulmonary fibrosis can be caused by various factors, for instance, long-term exposure to a number of toxins and pollutants (silica dusts, asbestos fibers, hard metal dusts, and bird and animal droppings, etc.), certain medical conditions (autoimmune diseases) and in some medications. Even so, the probable cause cannot be determined in most patients. Pulmonary fibrosis with no known cause is called idiopathic pulmonary fibrosis (IPF), which is an aging-related disease. The prevalence of this disease appears to be increasing. (5, 6) IPF occurs worldwide and is the most common of the idiopathic interstitial pneumonias with a median survival of less than 3-5 years following diagnosis. Predictors of a worse outcome of this disease include progressive dyspnea, oxygen desaturation, worsening pulmonary function and gas-exchange. In the reported animal models of pulmonary fibrosis, bleomycin (BLM)-induced pulmonary fibrosis in rats or mice is a conventional animal model. (7, 8)

It has been described that fibrosis can be treated by Signal Transducer and Activator of Transcription 3 (STAT3) inhibitors of various structural properties. (18)

Microcystins (MCs) are a common class of toxins produced by cyanobacterial blooms in various eutrophic inland waters. They are generally known to be inhibitors of serine/threonine protein phosphatases type 1 (PP1) and type 2A (PP2A) and thereby mainly produce hepatorenal toxicity. (9, 10) No related research has been reported so far on their biological activity against fibrosis and their application in developing clinical drugs. The main structure of MCs is a family of monocyclic heptapeptide compounds. Due to the difference in compositional amino acids, there are many isomers in nature, and the main variations occur in the second and fourth amino acids. The present disclosure first has selected the development of microcystins (MC-LR) in which the second and fourth amino acids of the monocyclic heptad structure are leucine (Leucine, L) and arginine (Arginine, R), respectively, and used MC-LR to prepare therapeutic and interventional drugs for use in treating pulmonary fibrosis. MC-LR has generally been described as potentially having toxic effects on alveolar type II epithelial cells and altering the renal parenchyma and renal physiology. (19, 20) Based on the effects observed with MC-LR, the present disclosure has further selected the development of microcystins (MC-RR) in which the second and fourth amino acids of the monocyclic heptad structure are arginine (Arginine, R). Therapeutic and interventional drugs have been prepared with MC-RR for pulmonary fibrosis and it was observed that the toxicity of MC-RR was significantly less than that of MC-LR. Toxicological studies on experimental mice (LD50) and cultured cells in *vitro* indicated that the toxicity of microcystin-RR was less than 1/5 of that of microcystin-LR. (10) Nonetheless, microcystin-RR still had good effects in treating and intervening pulmonary fibrosis.

Further described is constructing a bleomycin-induced rat pulmonary fibrosis model and then initiating the administration of microcystin-RR or microcystin-LR in drinking water to perform the intervention therapy. Following results gotten from the model animals: The treatment of microcystin-RR or microcystin-LR can alleviate and improve the pathological condition of bleomycin-induced pulmonary fibrosis, significantly reduce the content of Collagen 1 and inhibit expression of the alpha-smooth muscle actin (a-SMA) in the lung tissue of model rats. The expression of TGF-β, a critical molecule in fibrogenesis, and TGF-β/Smad signaling pathway proteins (Smad2/Smad3) are suppressed, while the expression of Smad7, an anti-fibrosis molecules, is up-regulated. The activation of TGF-β/Smad signaling pathway promotes epithelial to mesenchymal transition (EMT) and fibroblast to myofibroblast transition (FMT), which results in myofibroblast proliferation .(11-14) Microcystin-RR and microcystin-LR also inhibit the expression of P4HA3 (an α-subunit of collagen prolyl hydroxylase) in the model rats. P4HA3 is a TGF-β downstream target molecular and is involved in triple helical procollagen synthesis. (15) Additionally, an inhibited expression of vascular endothelial growth factor (VEGF) and endothelin 1 (ET-1) is associated with microcystin-RR and microcystin-LR treatment in the pulmonary fibrosis rats. Evidences show that VEGF facilitates fibrogenesis and ET-1 is able to induce mesenchymal cell mitosis. (16, 17)

The above experimental results, which are the basis of the present disclosure, clearly show that MC-RR and MC-LR have effective intervention and therapeutic effects on the pathological changes of pulmonary fibrosis induced by Bleomycin (BLM). Its important mechanism is to down-regulate the expression of TGF-β, suppress TGF-β/Smad signaling pathway, inhibit myofibroblast differentiation and collagen synthesis, and finally alleviate and block the abnormal deposition of extracellular matrix (ECM).

The present disclosure has found that microcystins as defined according to the claims can be used to develop and prepare novel drugs for preventing and treating organ tissue fibrotic diseases. In addition, the present disclosure also provides a method for inhibiting myofibroblast differentiation and collagen synthesis *in vitro* using microcystins as defined according to the claims, which can be used in scientific research, medical treatment and the like. It will be apparent that the above two aspects of the disclosure share a common technical feature that one or more of the microcystins that inhibit the TGF-β/Smad signaling pathway.

### SUMMARY OF THE DISCLOSURE

It is disclosed that microcystins (MCs), a monocyclic heptapeptide compound, is suitable to be used in medicine. Especially, microcystin-RR (MC-RR) and microcystin-LR (MC-LR) is applicable to prepare the drugs for the prevention or treatment of organ and tissue fibrosis diseases. The scope of the invention is defined by the claims.

According to the present disclosure the mentioned organ is lung.

Preferably, the medicament of the present disclosure blocks myofibroblast differentiation and inhibits the expression of α-SMA and Collagen 1 in the lung tissues of bleomycin-induced pulmonary fibrosis rats.

Preferably, the medicament of the present disclosure inhibits the expression of mRNA and protein of TGF-β gene in the lung tissues of bleomycin-induced pulmonary fibrosis rats.

Preferably, the medicament of the present disclosure inhibits the expression of Smad2 and Smad3 in the lung tissues of bleomycin-induced pulmonary fibrosis rats. Smad2 and Smad3 are the critical molecules of the TGF-/Smad signaling pathway. Activation of this signaling pathway promotes fibrogenesis.

Preferably, the medicament of the present disclosure up-regulates the expression of Smad7 in the lung tissues of bleomycin-induced pulmonary fibrosis rats. Smad7 is a negative protein of the TGF-/Smad signaling pathway. The increased expression of Smad7 inhibits fibrogenesis.

Preferably, the medicament of the present disclosure inhibits the expression of P4HA3 gene, a key downstream target molecular of TGF-β and involving in procollagen synthesis, in lung tissues of bleomycin-induced pulmonary fibrosis rats.

The present disclosure also discloses a method of inhibiting myofibroblast differentiation and collagen synthesis *in vitro* by using one or more microcystins as defined according to the claims to suppress the TGF-β/Smad signaling pathway. Preferably, in the *in vitro* method, the microcystin is a microcystin-LR containing a monocyclic heptad structure with the second and fourth amino acid residues being leucine and arginine, respectively, or microcystin-RR containing a monocyclic heptad structure with the second and fourth amino acid residuals both being arginine.

The disclosure is based on the classic animal model of bleomycin (BLM)-induced pulmonary fibrosis in rats (the animal model not forming part of the invention as such), which are then treated with MC-RR and MC-LR respectively. Based on histopathology and analysis of key signaling pathway molecules for fibrosis, MC-RR and MC-LR, the present disclosure proposes to alleviate and treat the pathological state of pulmonary fibrosis induced by BLM and to effectively prevent pulmonary fibrosis. Therefore, the present disclosure provides the molecular targets and mechanisms for using microcystin in the intervention and treatment of fibrosis. Natural microcystins (MCs) have multiple variants sharing the base structure of monocyclic heptapeptide but with different amino acid constituents, and the toxicity among different variants varies greatly. The present disclosure is based on the use of less toxic MC-RR and more toxic MC-LR intervention to treat pulmonary fibrosis in model rats, both of which show good intervention and therapeutic effects. It is proved that the anti-fibrotic effect of MCs is dependent on the molecular structure of monocyclic heptapeptide and on the resulting biological effects, but it does not rely on the toxicity of MCs per se.

Based on the above research, the present disclosure discloses that the microcystin monocyclic heptapeptide structure has the biological effect of relieving and blocking the pathogenesis of pulmonary fibrosis, and can be used for the development and preparation of a treatment and intervention drug for organ tissue fibrotic diseases.

### BRIEF DESCRIPTION OF THE DRAWINGS

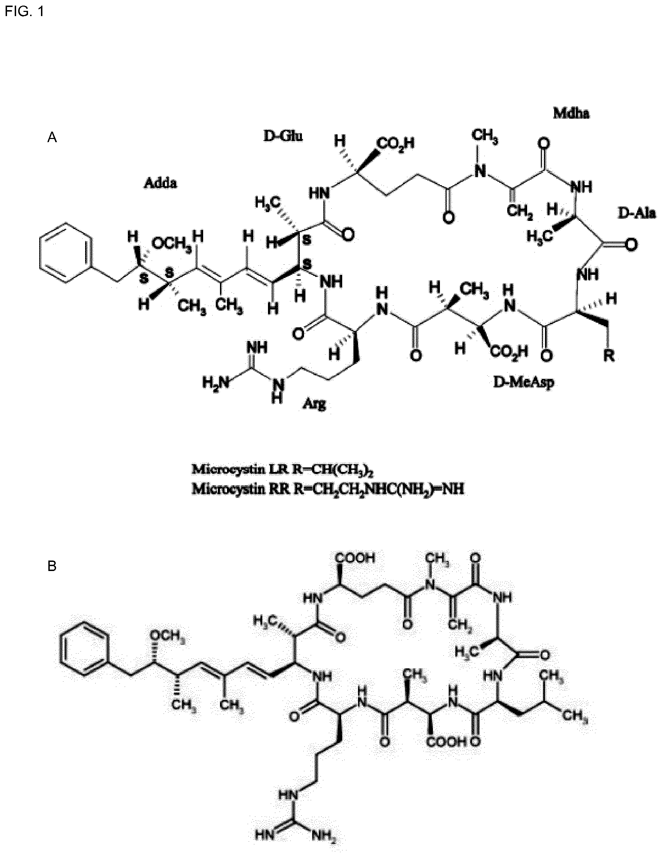

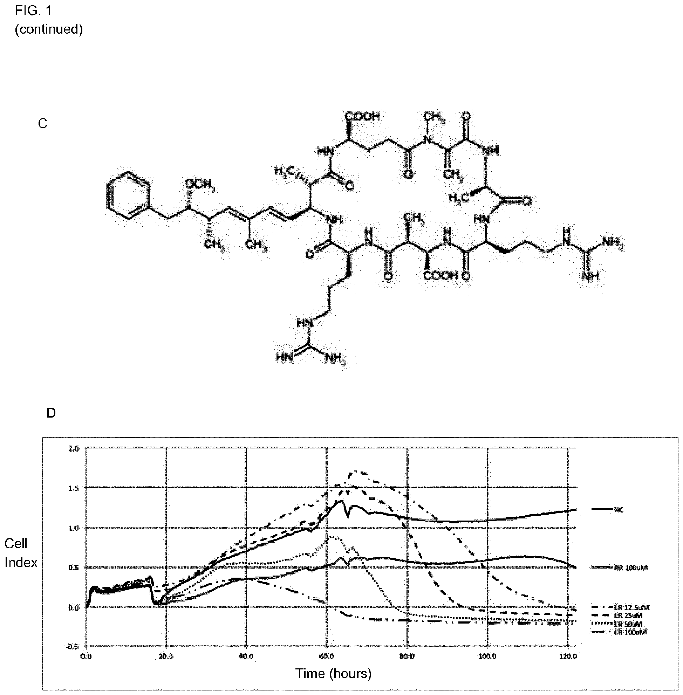

Figure 1 shows chemical structure of microcystins. A: Chemical structures of a monocyclic heptapeptide for microcystins; B: Chemical structures of microcystin-LR; C: Chemical structures of microcystin-RR; D: The test of toxic effects of microcystin-LR and microcystin-RR on cultured hepatocyte lines (L02) with real time cellular analysis (RTCA), showing that the cytotoxicity of microcystin-LR was significantly higher than that of microcystin-RR (NC: normal saline control).

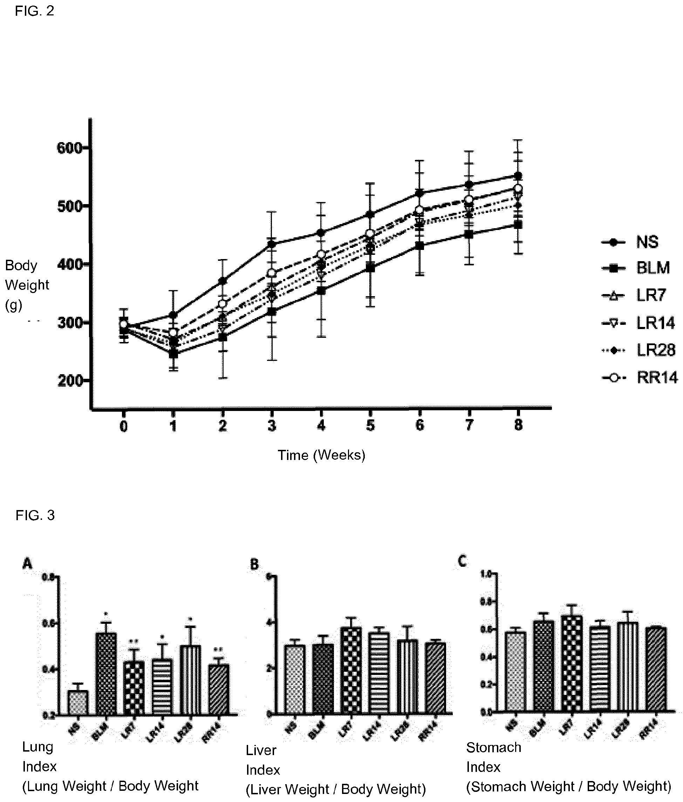

Figure 2 shows body weight changes of bleomycin-induced pulmonary fibrosis rats with or without microcystins treatment. NS: Normal saline control; BLM: The group of bleomycin (5.0 mg/kg) intratracheal instillation on day 0; LR7: The group of treatment of microcystin-LR (20 µg/L) in drinking water from day 7 (LR7); LR14: The group of treatment of microcystin-LR (20 µg/L) in drinking water from day 14; LR28: The group of treatment of microcystin-LR (20 µg/L) in drinking water from day 28; RR14: The group of treatment of microcystin-RR (20 µg/L) in drinking water from day 14. All rats of LR7, LR14, LR28 and RR14 were intratracheally instilled with bleomycin (5.0 mg/kg) on day 0.
Figure 3 shows organ coefficients of the experimental rats in different groups. A: The lung coefficient. ^{∗}Comparing with normal saline group; #Comparing with bleomycin (BLM) treatment group, P<0.05. B: The liver coefficient; C: The kidney coefficient.

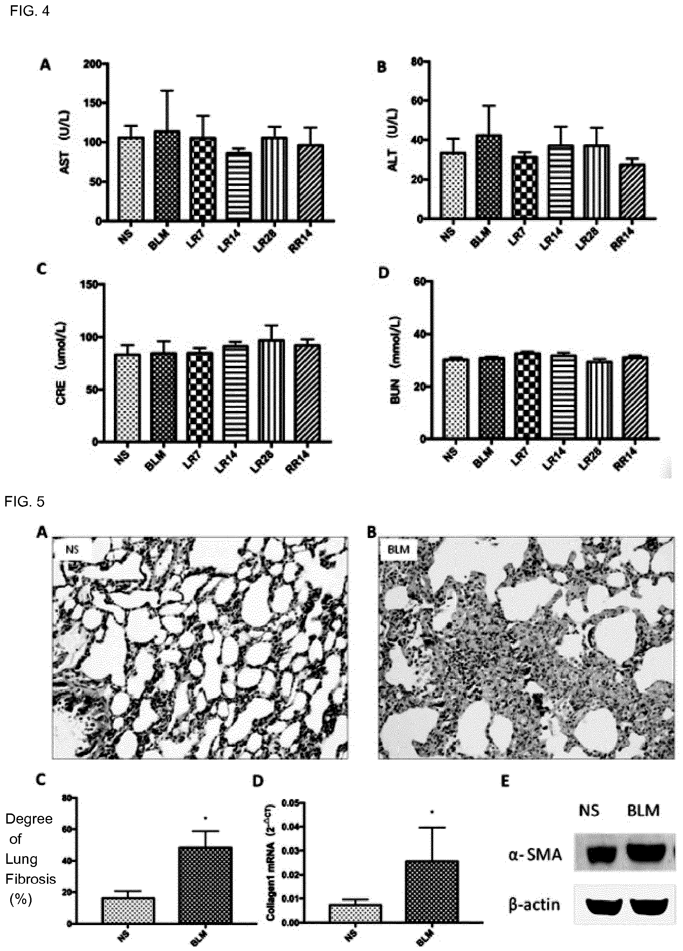

Figure 4 shows biochemical parameters of serum of the experimental rats in different groups. A and B: Serum levels of aspartate aminotransferase (AST) and alanine aminotransferase (ALT), reflecting liver function; C and D: Serum levels of creatinine (CRE) and urea nitrogen (BUN), reflecting renal function.
Figure 5 shows the rat pulmonary fibrosis induced with bleomycin. A and B: Lung tissue sections were subjected to Masson's trichrome staining from normal saline (NS) control (A) and from bleomycin intratracheal instillation (BLM) group (B); C: Comparison of pulmonary fibrosis scores between NS and BLM groups; D: Comparison on mRNA expression of collagen 1 between NS and BLM groups; E: Comparison on protein expression of α-SMA between NS and BLM groups. ^{∗} P<0.05.

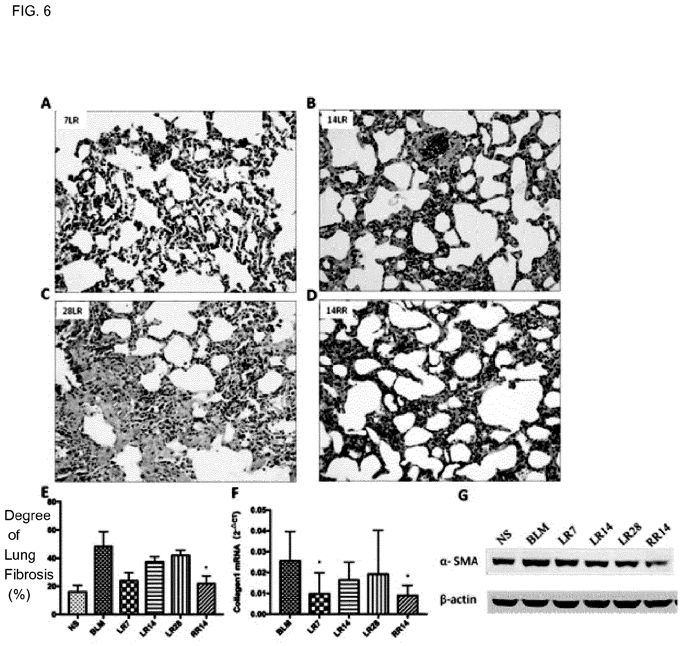

Figure 6 shows the effect of microcystin-RR (MC-RR) and microcystin-LR (MC-LR) on the bleomycin-induced rat pulmonary fibrosis. A-D: Lung tissue sections were subjected to Masson's trichrome staining from the experimental rats. The treatment of microcystins ameliorated pulmonary fibrosis in LR7 (A), LR14 (B), LR28 (C) and RR-14 (D) groups, respectively. E: Comparison on pulmonary fibrosis scores among the treated groups with microcystin-RR or microcystin-LR and BLM group, which showed the treatment of microcystin-RR and microcystin-LR ameliorated bleomycin-induced pulmonary fibrosis, especially in LR7 and RR14 groups. F: The mRNA expression of collagen 1, showing a significantly down-regulation in LR7 and RR14 groups. G: A inhibited protein expression of α-SMA is associated with treatment of microcystin-LR and microcystin-RR, especially in LR28 and RR14 groups. ^{∗} Comparing with BLM group, P<0.05.

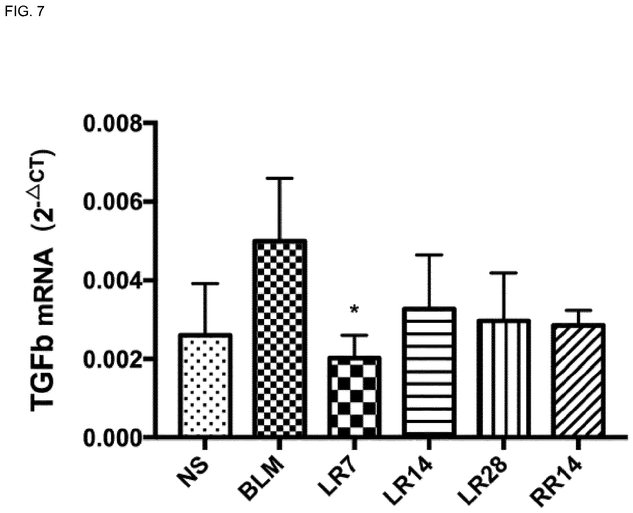

Figure 7 shows that the treatment of microcystin-RR or microcystin-LR inhibits mRNA expression of TGF-β in the lung tissues of bleomycin-induced pulmonary fibrosis rats. ^{∗} Comparing with BLM group, P<0.05.

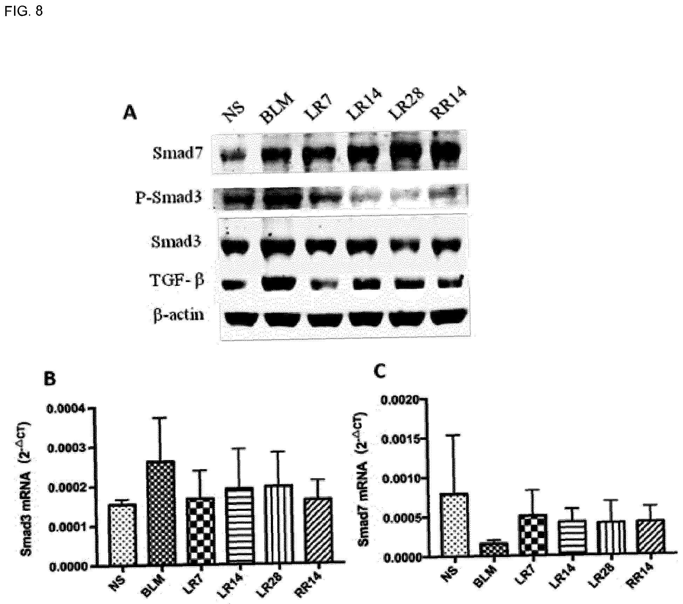

Figure 8 shows that the treatment of microcystin-RR or microcystin-LR suppresses the TGF-/Smad signaling pathway in the lung tissues of bleomycin-induced pulmonary fibrosis rats. A: The expression changes of the proteins in TGF-/Smad signaling pathway with microcystin-RR or microcystin-LR treatment. B: The transcriptional expression of Smad3 gene in the lung tissues of different groups. C: The transcriptional expression of Smad7 gene in the lung tissues of different groups.

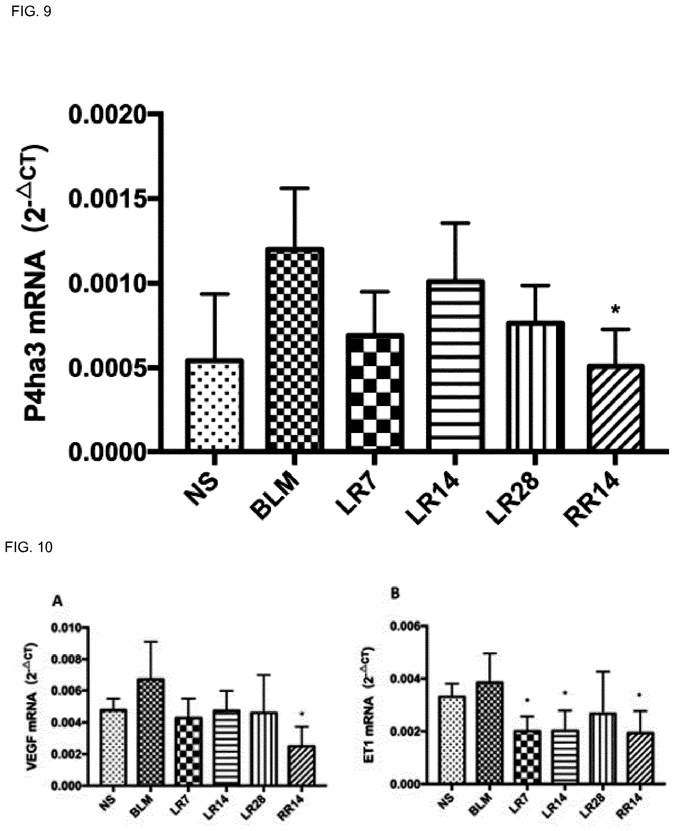

Figure 9 shows that the treatment of microcystin-RR or microcystin-LR inhibits mRNA expression of P4HA3 gene in lung tissue of bleomycin-induced pulmonary fibrosis rats. P4HA3 is a key downstream target molecular involved in procollagen synthesis. ^{∗} Comparing with BLM group, P<0.05.
Figure 10 shows that the treatment of microcystin-RR or microcystin-LR inhibits mRNA expression of VEGF and ET-1, both genes associated with fibrogenesis, in lung tissues of bleomycin-induced pulmonary fibrosis rats. ^{∗} Comparing with BLM group, P<0.05.

### DETAILED DESCRIPTION OF THE DISCLOSURE WITH EMBODIMENTS

The bleomycin (BLM)-induced pulmonary fibrosis in rats (or mice) is a universally accepted animal model used by laboratories to study the mechanism of pulmonary fibrosis, intervention targets and therapeutic drugs. This model establishes well-defined histopathological changes which are very similar to human pulmonary fibrosis. The rat BLM exposure method used was a commonly used orotracheal intubation, instilled in the airway; and the control was intratracheally instilled with an equal volume of saline.

Experimental animals: SPF grade Sprague Dawley (SD) rats, male, weighing 200g-250g, provided by Changzhou Cavans Laboratory Animal Co., Ltd.(animal qualified number: SCXK (Su) 2011-0003). Breeding conditions: a well-ventilated environment, at 20 °C constant temperature, under a 12hrs light/dark cycle, with free access to drinking water and food. Pulmonary fibrosis model: induced by Bleomycin (BLM, Nippon Kayaku Co., Ltd.) with SD male rats, approximately 8 weeks old. The rats, anesthetized with 10% chloral hydrate (0.4 ml/kg), were intratracheally instilled with a single dose of bleomycin (5mg/kg). The control rats were given equal volume saline by intratracheal instillation.

A total of 36 SD rats were randomly divided into six groups (6 per group): [1] normal saline control group (NS); [2] bleomycin-induced pulmonary fibrosis model (BLM); [3] the rats that were intratracheally instilled with a single dose of bleomycin and then treated with microcystin-LR (20 µg/L) in drinking water starting on day 7 (LR7); [4] the rats that were intratracheally instilled with a single dose of bleomycin and then treated with microcystin-LR (20 µg/L) in drinking water starting on day 14 (LR14); [5] the rats that were intratracheally instilled with a single dose of bleomycin and then treated with microcystin-LR (20 µg/L) in drinking water starting on day 28 (LR28); [6] the rats that were intratracheally instilled with a single dose of bleomycin and then treated with microcystin-RR (20 µg/L) in drinking water starting on day 14 (RR14). All animals were sacrificed on day 56 after bleomycin intratracheal instillation. The blood samples were obtained to isolate serum and tissues samples were taken from lung, liver and kidney. Lung tissue samples were taken from the lobes of the lungs. The tissue samples were each divided into two portions, one fixed in 4% paraformaldehyde and the other frozen in liquid nitrogen. The paraformaldehyde fixed samples were used for histopathological analysis, and liquid nitrogen preserved samples were used for protein and nucleic acid analysis. Statistical evaluation of the data was performed with SPSS 11.5. One-way analysis of variance (ANOVA) was applied for calculating the significance of the difference between the control group and the treatment groups. Statistical significance was set at P< 0.05.

### Experimental Results

The intervention and therapeutic effects of microcystin-RR and microcystin-LR on bleomycin-induced pulmonary fibrosis were investigated in the present disclosure. The results are as follows:
(1) MC-RR or MC-LR did not produce significant toxic effects on the model rats by dose and mode of administration as used in the experiments. (i) Compared with saline control group, bleomycin-induced rats (containing the groups with microcystin-RR or microcystin-LR treatment) had a severe body weight loss on the first week after bleomycin intratracheal instillation. However, the administration of microcystin-RR or microcystin-LR improved the weight recovery among BLM-induced rats (Figure 2). No difference of the organ indexes for liver and kidney among bleomycin-exposed rats with and without microcystin-LR or microcystin-RR treatment, but microcystin-RR and microcystin-LR can attenuate bleomycin-induced increasing of lung indexe (Figure 3). (ii) Microcystin-RR and microcystin-LR used in this work did not affect the biochemical parameters of serum for liver and kidney function (Figure 4).
(2) The rat model of pulmonary fibrosis was constructed by intratracheal instilling with a single dose of bleomycin (5mg/kg). (i) The lesions of pulmonary fibrosis were revealed by Masson's trichrome staining of lung tissues sections. The results showed that the lung tissue of BLM model group showed obvious fibrosis state. (ii) The expression of collagen protein (mRNA transcription) in lung tissue of BLM group was significantly higher than that of normal saline control group. (iii) The expression of α-SMA (a-smooth muscle actin) protein was significantly increased, reflecting the proliferation of myofibroblasts in the lung tissue of rats in the BLM group (Figure 5).
(3) Microcystin-RR and microcystin-LR significantly ameliorate bleomycin-induced pulmonary fibrosis. (i) The lung tissue sections with Masson's trichrome staining showed that the treatment of Microcystin-RR or microcystin-LR reduced fibrosis lesions in bleomycin-induced pulmonary fibrosis rats, significantly in RR14, LR7 and LR 14 groups. (ii) Compared with bleomycin-induced rats (BLM group), the expression of collagen 1 in the lung tissues was down-regulated at the transcriptional level following microcystin-RR or microcystin-LR treatment, especially in RR14 and LR7 groups. (iii) Compared with bleomycin-induced rats (BLM group), the expression of α-SMA in the lung tissues was down-regulated at translational level following microcystin-RR or microcystin-LR treatment (Figure 6).
(4) The intervention of MC-RR or MC-LR can significantly inhibit the expression of TGF-β in rat lung tissue induced by BLM. TGF-β is the most potent protein factor so far known to promote tissue fibrosis, and it is also a mechanism of fibrosis formation and an important molecular target for anti-fibrosis research. TGF-β promotes epithelial-mesenehymal transition (EMT) and myofibroblast formation. (11, 12) Intratracheal instillation of BLM caused a significant increase in the release of TGF-β from rat lung tissue cells. Following instillation of BLM in the airway, the intervention with MC-RR or MC-LR significantly inhibited the transcription and translation of TGF-β. The LR7 intervention group and RR14 intervention group had the greatest inhibition of TGF-β expression (Figure 7 and Figure 8A).
(5) Microcystin-RR and microcystin-LR significantly suppress the activity of TGF-β/Smad signaling pathway in the lung tissues of bleomycin-induced pulmonary fibrosis rats. TGF-β released from tissue cells, by binding on the TGF-β receptor on the surface of cell membrane, activates the TGF-β/Smad signaling pathway. It causes an increase in Smad2/3 expression (increased protein phosphorylation) and forms a complex with Smad4, which is transferred into the nucleus, alters nuclear gene expression, initiates EMT and myofibroblast differentiation, leading to tissue fibrosis . (13, 14) In the lung tissues of bleomycin-induced pulmonary fibrosis rats, the expressions of Smad2/3 (including phosphorylated Smad2/3) were significantly up-regulated, indicating activation of TGF-β/Smad signaling pathway. Drinking water MC-RR or MC-LR intervention of the BLM-model rats significantly reduced Smad2/3 expression (including decreased protein phosphorylation) in tissue cells. TGF-β/Smad signaling pathway in normal cells is subject to the inhibition by Smad7. Rats intervened with MC-RR or MC-LR showed a significant increase in Smad7 expression. Those experimental data sufficiently demonstrate the inhibitory effect of MC-RR and MC-LR on bleomycin-induced induced pulmonary fibrosis (Figure 8).
(6) Microcystin-RR and microcystin-LR significantly suppressed the expression of P4HA3, an □-subunit of collagen prolyl hydroxylase, in lung tissues of bleomycin-induced pulmonary fibrosis rats. P4HA3 was recently identified as a key downstream target gene of TGF-β, involved in procollagen synthesis. An up-regulated expression of P4HA3 has been observed in the lung tissues of pulmonary fibrosis patients and bleomycin-induced pulmonary fibrosis. (15) The treatment of microcystin-RR or microcystin-LR can inhibit the expression of P4HA3, especially in the rats of RR14 and LR7 groups (Figure 9).
(7) Microcystin-RR and microcystin-LR significantly suppressed the expressions of VEGF and ET1, both cytokines promoting the formation of fibrosis. (16) The pathological development of fibrosis is often associated with an up-regulation of VEGF and ET-1 expressions. VEGF is one of the most potent vascular growth factors. Increased levels of VEGF in tissues can promote vascular permeability, leading to macrophage migration and aggregation, which cause the TGF-β release. ET-1 can promote fibroblast proliferation, induce fibroblast to myofibroblast transition (FMT) and inhibit collagen degradation. (17) The treatment of microcystin-RR or microcystin-LR significantly decreased the transcriptional levels VEGF and ET-1 genes (Figure 10).

The above experimental results, which are the basis of the presented disclosure, clearly show that MC-RR and MC-LR have effective intervention and therapeutic effects against the pathological changes of bleomycin-induced pulmonary fibrosis. Its important mechanism is to down-regulate the expression of TGF-β, suppress TGF-β/Smad signaling pathway, inhibit myofibroblast differentiation and collagen synthesis, and finally alleviate and block the abnormal deposition of extracellular matrix (ECM).

Therefore, the microcystins provided by the present disclosure have novel and important uses in the development and preparation of drugs for preventing and treating organ tissue fibrotic diseases.

### References

1. Wallace K, Burt AD, Wright MC. Liver fibrosis. Biochem J, 2008, 411(1):1.
2. LeBleu VS, Taduri G, O'Connell J, Teng Y, Cooke VG, Woda C, Sugimoto H, Kalluri R. Origin and function of myofibroblasts in kidney fibrosis. Nat Med, 2013, 19(8): 1047-53.
3. Meng XM, Tang PM, Li J, Lan HY. TGF-β/Smad signaling in renal fibrosis. Front Physiol, 2015, 6:82.
4. Hutchinson J, Fogarty A, Hubbard R, McKeever T. Global incidence and mortality of idiopathic pulmonary firosis: a systematic review. Eur Respir, 2015, 46(3):795.
5. Baumgartner KB, Samet JM, Coultas DB, Stidley CA, Hunt WC, Colby TV, Waldron JA. Occupational and environmental risk factors for idiopathic pulmonary firosis: a multicenter case-control study. Am J Epidemiol, 2000, 152(4):307.
6. King TJ, Pardo A, Selman M. Idiopathic pulmonary fibrosis. Lancet, 2011, 378( 9807):1949.
7. Tager AM, LaCamera P. Shea BS, Campanella GS, Selman M, Zhao Z, Polosukhin V, Wain J, Karimi-Shah B A, Kim ND, Hart WK, Pardo A, Blackwell TS, Xu Y, Chun J, Luster AD. The lysophosphatidic acid receptor LPA1 links pulmonary fibrosis to lung injury by mediating fibroblast recruitment and vascular leak. Nat Med, 2008, 14:45.
8. Zhang H, Liu XF, Chen S, Wu JH, Ye X, Xu LZ, Chen HM, Zhang DP, Tan RX, Wang YP, Tectorigenin inhibits the in vitro proliferation and enhances miR-338∗ expression of pulmonary fibroblasts in rats with idiopathic pulmonary fibrosis. J Ethnopharmacol, 2010, 131(1):165.
9. van Apeldoorn ME, van Egmond HP, Speijers GJ, Bakker GJ. Toxins of cyanobacteria. Mol Nutr Food Res, 2007, 51(1):7-60
10. Wolf HU, Frank C. Toxicity assessment of cyanobacterial toxin mixtures. Environ Toxicol, 2002, 17, 395-399.
11. Allison SJ. Fibrosis: the source of myofibroblasts in kidney fibrosis. Nat. Rev. Nephrol, 2013, 9:494.
12. Kalluri R, Neilson EG Epithelial-mesenchymal transition and its implications for fibrosis. J Clin Invest, 2003, 112:1776.
13. Lan HY. Diverse roles of TGF-β/Smads in renal fibrosis and inflammation. Int J Biol Sci, 2011, 7(7):1056.
14. Yan X, Liu Z, Chen Y. Regulation of TGF-beta signaling by Smad7. Acta Biochim Biophys Sin (Shanghai), 2009, 41(4):263.
15. Luo Y, Xu W, Chen H, Warburton D, Dong R, Qian B, Selman M, Gauldie J, Kolb M, Shi W. A novel profibrotic mechanism mediated by TGF-β-stimulated collagen prolyl hydroxylase expression in fibrotic lung mesenchymal cells. J Pathol, 2015, 236(3): 384.
16. Wernig G, Chen SY, Cui L, Van Neste C, Tsai JM, Kambham N, Vogel H, Natkunam Y, Gilliland DG, Nolan G, Weissman IL. Unifying mechanism for different fibrotic diseases. Proc Natl Acad Sci USA, 2017, 114(18):4757.
17. Anguiano L, Riera M, Pascual J, Soler MJ. Endothelin Blockade in Diabetic Kidney Disease. J Clin Med, 2015, 4(6):1171.
18. WO 2009/059425, STA3 Inhibitors for the Treatment of Fibrosis (The University of British Columbia)
19. Wang C, Gu Shen, Yin X, Yuan M, Xiang Z, Li Z, Cao H, Meng X, Hu K, Han X. The toxic effects of microcystin-LR on mouse lungs and alveolar type II epithelial cells. Toxicon, 2016, 115:81-88.
20. Lowe J, Souza-Menezes J, Freire DS, Mattos LJ, Castiglione RC, Barbosa CM, Santiago L, Ferrão FM, Cardoso LH, da Silva RT, Vieira-Beiral HJ, Vieyra A, Morales MM, Azevedo SM, Soares RM. Single sublethal dose of microcystin-LR is responsible for different alterations in biochemical, histological and physiological renal parameters. Toxicon, 2012, 59(6):601-609.

## Claims

1. A microcystin for use in preventing or treating of fibrosis disease, wherein the microcystin either is a monocyclic heptapeptide compound with L-leucine and L-arginine residues at positions 2 and 4 of the compound, respectively, or a monocyclic heptapeptide compound with L-arginine residuals at both positions 2 and 4, and wherein the fibrosis disease is a lung fibrosis disease.

2. The microcystin for use according to claim 1, wherein the microcystin is used for inhibiting the formation of myofibroblasts in the prevention of fibrosis tissue generation.

3. The microcystin for use according to claim 1, wherein the microcystin is used for reducing the expression level of α-SMA in fibrotic lung tissue in the treatment of pulmonary fibrosis disease.

4. The microcystin for use according to claim 1, wherein the microcystin is used for inhibiting the expression of Collagen 1 in fibrotic lung tissue in the treatment of pulmonary fibrosis disease.

5. The microcystin for use according to claim 1, wherein the microcystin is used for inhibiting the expression of mRNA and protein of TGF-β gene in lung tissue in the prevention of pulmonary fibrosis disease.

6. The microcystin for use according to claim 1, wherein the microcystin is used for inhibiting the expression of Smad2 and Smad3 genes in lung tissue in the prevention of pulmonary fibrosis disease.

7. The microcystin for use according to claim 1, wherein the microcystin is used for up-regulating the expression of Smad7 gene in lung tissue in the prevention of pulmonary fibrosis disease.

8. The microcystin for use according to claim 1, wherein the microcystin is used for inhibiting the expression of P4HA3 gene in fibrotic lung tissue in the treatment of pulmonary fibrosis disease.

9. A method for inhibiting myofibroblast differentiation and collagen synthesis *in vitro,* comprising blocking the TGF-β/Smad signalling pathway with a microcystin that either is a monocyclic heptapeptide compound with L-leucine and L-arginine residues at positions 2 and 4 of the compound, respectively, or a monocyclic heptapeptide compound with L-arginine residuals at both positions 2 and 4.

## Patentansprüche

1. Mikrocystin zur Verwendung beim Vorbeugen oder Behandeln einer Fibroseerkrankung, wobei das Mikrocystin entweder eine monozyklische Heptapeptid-Verbindung mit L-Leucin-und L-Argininresten an den Positionen 2 beziehungsweise 4 der Verbindung oder eine monozyklische Heptapeptid-Verbindung mit L-Argininresten an beiden Positionen 2 und 4 ist, und wobei die Fibroseerkrankung eine Lungenfibroseerkrankung ist.

2. Mikrocystin zur Verwendung gemäß Anspruch 1, wobei das Mikrocystin zur Hemmung der Bildung von Myofibroblasten bei der Vorbeugung der Bildung von Fibrosegewebe verwendet wird.

3. Mikrocystin zur Verwendung gemäß Anspruch 1, wobei das Mikrocystin zur Verringerung des Expressionsniveaus von α-SMA in fibrotischem Lungengewebe bei der Behandlung von pulmonaler Fibroseerkrankung verwendet wird.

4. Mikrocystin zur Verwendung gemäß Anspruch 1, wobei das Mikrocystin zur Hemmung der Expression von Kollagen 1 in fibrotischem Lungengewebe bei der Behandlung von pulmonaler Fibroseerkrankung verwendet wird.

5. Microcystin zur Verwendung gemäß Anspruch 1, wobei das Microcystin zur Hemmung der Expression von mRNA und Protein des TGF-β-Gens in Lungengewebe bei der Vorbeugung von pulmonaler Fibroseerkrankung verwendet wird.

6. Microcystin zur Verwendung gemäß Anspruch 1, wobei das Microcystin zur Hemmung der Expression der Smad2- und Smad3-Gene in Lungengewebe bei der Vorbeugung von pulmonaler Fibroseerkrankung verwendet wird.

7. Mikrocystin zur Verwendung gemäß Anspruch 1, wobei das Mikrocystin zur Hochregulierung der Expression des Smad7-Gens in Lungengewebe bei der Vorbeugung von pulmonaler Fibroseerkrankung verwendet wird.

8. Microcystin zur Verwendung gemäß Anspruch 1, wobei das Microcystin zur Hemmung der Expression des P4HA3-Gens in fibrotischem Lungengewebe bei der Behandlung von pulmonaler Fibroseerkrankung verwendet wird.

9. Verfahren zum Hemmen der Myofibroblasten-Differenzierung und der Kollagensynthese in *vitro,* umfassend das Blockieren des TGF-β/Smad-Signalwegs mit einem Microcystin, das entweder eine monozyklische Heptapeptid-Verbindung mit L-Leucin- und L-Argininresten an den Positionen 2 beziehungsweise 4 der Verbindung oder eine monozyklische Heptapeptid-Verbindung mit L-Argininresten an beiden Positionen 2 und 4 ist.

## Revendications

1. Mycrocystine pour une utilisation dans la prévention ou le traitement d'une maladie de type fibrose, la mycrocystine étant soit un composé de type heptapeptide monocyclique comportant des résidus de L-leucine et de L-arginine au niveau des positions respectivement 2 et 4 du composé, soit un composé de type heptapeptide monocyclique comportant des résidus de L-arginine à la fois au niveau des positions 2 et 4, et la maladie de type fibrose étant une maladie de type fibrose du poumon.

2. Mycrocystine pour une utilisation selon la revendication 1, la mycrocystine étant utilisée pour inhiber la formation de myofibroblastes dans la prévention de la génération tissulaire de fibrose.

3. Mycrocystine pour une utilisation selon la revendication 1, la mycrocystine étant utilisée pour réduire le niveau d'expression de α-SMA dans un tissu de poumon fibrotique dans le traitement d'une maladie de type fibrose pulmonaire.

4. Mycrocystine pour une utilisation selon la revendication 1, la mycrocystine étant utilisée pour inhiber l'expression de Collagène 1 dans un tissu de poumon fibrotique dans le traitement d'une maladie de type fibrose pulmonaire.

5. Mycrocystine pour une utilisation selon la revendication 1, la mycrocystine étant utilisée pour inhiber l'expression d'ARNm et de protéine du gène TGF-β dans un tissu de poumon dans la prévention d'une maladie de type fibrose pulmonaire.

6. Mycrocystine pour une utilisation selon la revendication 1, la mycrocystine étant utilisée pour inhiber l'expression de gènes Smad2 et Smad3 dans un tissu de poumon dans la prévention d'une maladie de type fibrose pulmonaire.

7. Mycrocystine pour une utilisation selon la revendication 1, la mycrocystine étant utilisée pour réguler à la hausse l'expression du gène Smad7 dans un tissu de poumon dans la prévention d'une maladie de type fibrose pulmonaire.

8. Mycrocystine pour une utilisation selon la revendication 1, la mycrocystine étant utilisée pour inhiber l'expression du gène P4HA3 dans un tissu de poumon fibrotique dans le traitement d'une maladie de type fibrose pulmonaire.

9. Procédé pour l'inhibition de la différenciation de myofibroblastes et de la synthèse de collagène in vitro, comprenant le blocage de la voie de signalisation TGF-β/Smad avec une mycrocystine qui est soit un composé de type heptapeptide monocyclique comportant des résidus de L-leucine et de L-arginine au niveau des positions respectivement 2 et 4 du composé, soit un composé de type heptapeptide monocyclique comportant des résidus de L-arginine à la fois au niveau des positions 2 et 4.
